# EUROPEAN PATENT APPLICATION

(11) **EP 0 761 300 A2**
(43) Date of publication of application: **12.03.1997**
(21) Application number: 96305960.5
(22) Date of filing: 15.08.1996
(51) Int. Cl.: B01J 19/00, C07C 43/04, C07C 41/09

(54) **Feed control in the manufacture of methyl tertiary butyl ether**

(30) Priority: 18.08.1995 US 19884
(71) Applicant: TEXACO DEVELOPMENT CORPORATION, White Plains, New York 10650 (US)
(72) Inventor: Parker, Stuart John, Houston, Texas 77005 (US); Mijares, Gerardo, Woodlands, Texas 77381 (US); Hwan, Rei-Yu Judy, Sugar Land, Texas 77479 (US)
(74) Representative: Green, Mark Charles

(57) **Abstract**

A first stream of predeterminable temperature is mixed with a second stream of a different predeterminable temperature to provide a final mixed stream by flowing a portion of the second stream through a by-pass indirect contact heat exchanger into indirect heat exchange contact with a flowing stream of a heat exchange liquid, by-passing a second portion of the second stream, by mixing the first and second portions to provide a reconstituted second stream having an intermediate temperature, by mixing the first stream with the reconstituted second stream to provide a final stream having a final temperature and by changing the portion of the second stream flowing through the by-pass in response to a change in the final temperature by an amount sufficient to maintain the temperature of the final stream, for example, in the preparation of a charge stream used in the preparation of methyl tertiary butyl ether from tertiary butyl alcohol and methanol.

## Description

### Technical Field of the Invention

This invention relates to a process for the preparation of a charge stream for use in the continuous preparation of methyl tertiary butyl ether (MTBE) from tertiary butyl alcohol (TBA) and methanol (MeOH) wherein the temperatures of a MeOH stream, a higher boiling distillation fraction and a peroxides-free TBA stream are adjusted and wherein the streams are then mixed in proportions to provide a charge stream having a preselected predeterminable charge temperature within a predetermined reaction temperature range.

In accordance with the present invention, three streams are mixed, providing a final stream of a desired temperature, by flowing a portion of a primary stream through a first by-pass indirect contact heat exchanger, flowing a second portion of the stream through the by-pass of the heat exchanger and mixing the portions to provide a reconstituted primary stream having an intermediate temperature, flowing a portion of a secondary stream through a second by-pass indirect contact heat exchanger, flowing a second portion through the heat exchanger, and mixing the portions to provide a reconstituted secondary stream having an intermediate temperature, mixing the reconstituted primary and secondary streams with a third stream to provide a final stream having a desired final temperature, and changing the portion of the primary or secondary stream flowing through a by-pass of the corresponding by-pass indirect contact heat exchanger in response to a change in the temperature of the final mixed stream.

### Prior Art

The preparation of methyl tert-butyl ether from methyl and tert-butyl alcohols is discussed in S. V. Rozhkov et al., Prevrashch Uglevodorodov, Kislotno-Osnovn. Geterogennykh Katal. Tezisy Dokl., Vses. Konf., 1977, 150 (C. A. 92:58165y). Here the TBA and methanol undergo etherification over KU-2 strongly acidic sulfopolystyrene cation-exchangers under mild conditions. This reference contains data on basic parameters of such a process.

US-A-5,243,091 discloses a method for the preparation of methyl tertiary butyl ether wherein a mixture of methanol and tertiary butyl alcohol is catalytically reacted to form a reaction product that is separated into a first lower boiling distillation fraction comprising isobutylene, methanol and methyl tertiary butyl ether and a first higher boiling distillation fraction comprising methanol, tertiary butyl alcohol and water. The first higher boiling distillation fraction is distilled to provide a second lower boiling TBA fraction for recycle. The first lower boiling distillation fraction and isobutylene are reacted in a finishing reactor to form a finishing reactor conversion product that is charged to a methanol extraction zone and countercurrently contacted with water to provide an overhead extract comprising MTBE, water and isobutylene, from which the isobutylene is recovered for recycle.

US-A-5,292,964 discloses a method for the preparation of methyl tertiary butyl ether wherein a mixture of methanol and tertiary butyl alcohol are catalytically reacted to form a reaction product containing the water of etherification and at least one mol of methanol per two moles of methyl tertiary butyl ether, the reaction product being fractionated to separate a lower boiling methanol and methyl tertiary butyl ether fraction from the water and tertiary butyl alcohol and wherein the methanol in the lower boiling distillation fraction is reacted with isobutylene to form additional methyl tertiary butyl ether.

This invention relates to a process for the preparation of a final charge stream for use in the continuous preparation of methyl tertiary butyl ether (MTBE) from tertiary butyl alcohol (TBA) and methanol (MeOH), the final charge stream having a desired final predeterminable temperature within a predetermined temperature range, including the steps of
a) flowing a higher boiling distillation fraction from an MTBE distillation zone through a first by-pass indirect contact heat exchanger into indirect heat exchange contact with a flowing stream comprising a predetermined portion of a lower boiling distillation recycle fraction from a recycle distillation column while flowing the remainder of the lower boiling distillation recycle fraction through the by-pass of the first by-pass indirect contact heat exchanger, and recombining the portions of the lower boiling distillation recycle fraction to form a reconstituted lower boiling distillation recycle fraction having a desired lower temperature,
b) flowing a selected portion of a second stream through the by-pass of a second by-pass indirect contact heat exchanger and flowing the remainder of the second stream through the second by-pass indirect contact heat exchanger in indirect heat exchange contact with a peroxides-contaminated TBA feedstock and recombining the portions downstream of the second by-pass indirect contact heat exchanger to form a reconstituted second stream having a desired lower temperature,
c) mixing the reconstituted lower boiling distillation recycle fraction and the reconstituted second stream with a MeOH stream to provide the final charge stream having the desired final predeterminable temperature within the predetermined temperature range, and
d) changing the portion of at least one of the second stream or the lower boiling distillation recycle fraction flowing through a by-pass of the first or second by-pass indirect contact heat exchangers in response to a change in the temperature of the final charge stream by an amount sufficient to maintain the final charge stream at a temperature within the predetermined temperature range.

### The Etherification Reaction Catalyst

In accordance with the MTBE manufacture and purification method of the present invention, a primary etherification reactor containing a bed of etherification catalyst is utilized. A wide variety of etherification catalysts can be used for this purpose, such as supported phosphorus acid-type catalysts. A preferred catalyst is an acid cation exchange resin such as an acidic ion exchange resin consisting essentially of sulfonated polystyrene, for example, a divinyl benzene crosslinked polystyrene matrix containing from 0.5 to 20% of copolymerized divinyl benzene. Resins of this nature are manufactured and sold commercially under various trade names such as "Dowex 50", "Nalcite HCR" and "Amberlyst 15". The use of catalyst of this nature is disclosed, for example, in US-A-4,144,138.

Also, Kieselguhr impregnated with phosphoric acid as disclosed in US-A-2,282,469, titania having phosphoric acid impregnated thereon as disclosed in US-A-4,822,921, a hetero polyacid such as 12-tungstophosphoric acid or 12-molybdophosphoric acid supported on titania may be used.

Zeolites as disclosed in JP-B-0007432 or aluminosilicate zeolites as disclosed in US-A-4,058,576 may also be used.

The reaction conditions to be utilized when reacting methanol with tertiary butyl alcohol in the presence of a sulfonic acid resin etherification catalyst include a reaction temperature of 90° to 140°C, a pressure of 207 to 3450 kPa (30 to 500 psia) and a space velocity of 0.5 to 20 volumes of feed per volume of catalyst per hour.

### Peroxide Decomposition

The tertiary butyl alcohol feedstock to be used in the preparation of methyl tertiary butyl ether in accordance with the present invention is a tertiary butyl alcohol feedstock contaminated with peroxides such as tertiary butyl hydroperoxide, ditertiary butyl peroxide, allyl tertiary butyl peroxide. The feedstock is treated for the substantially complete removal of the peroxide contaminants before it is charged to the methyl tertiary butyl ether etherification zone.

It is known to prepare tertiary butyl alcohol by the thermal or catalytic decomposition of tertiary butyl hydroperoxide. It is also known to prepare tertiary butyl alcohol by the catalytic reaction of tertiary butyl hydroperoxide with propylene to form propylene oxide and tertiary butyl alcohol. The tertiary butyl alcohol feedstock derived from tertiary butyl hydroperoxide in this manner will contain peroxide contaminants. A typical feedstock prepared in this fashion will contain from 95 to 99 wt.% of tertiary butyl alcohol and less than 2.0 wt.% of peroxide contaminants.

In accordance with the present invention, the peroxides-contaminated tertiary butyl alcohol is charged to a peroxides decomposition reaction zone where the peroxides are substantially completely thermally and/or catalytically decomposed. The peroxide contaminants will be decomposed to form water and tertiary butyl alcohol, and trace amounts of other decomposition products such as acetone and methyl formate.

The peroxides-contaminated tertiary butyl alcohol feedstock is continuously passed through a peroxides decomposition reactor at a temperature of 100° to 200°C., a pressure of 550 to 3450 kPa (80 to 500 psia) at a flow rate of 0.5 to 20 volumes of feedstock per reactor volume per hour to thereby provide a substantially peroxides-free tertiary butyl alcohol reaction product.

Alternately, the peroxide contaminants may be catalytically decomposed.

A wide variety of catalysts may be used for this purpose, such as cobalt borate as disclosed in US-A-4,547,598, a nickel, copper, chromia catalyst as disclosed in US-A-4,704,482, an iron, copper, chromia, cobalt catalyst as disclosed in US-A-4,705,903, a base treated hydrogenation catalyst from groups VIB or VIIIB of the Periodic Table as disclosed in US-A-4,742,179, a nickel, copper, chromium and barium catalyst as disclosed in US-A-4,873,380, a metal phthalocyanine catalyst as disclosed in US-A-4,910,349, an imidazole-promoted methyl metal phthalocyanine catalyst as disclosed in US-A-4,912,266, a base promoted metal phthalocyanine catalyst as disclosed in US-A-4,912,267, a solid ruthenium catalyst as disclosed in US-A-4,922,033, a promoted metal porphine catalyst as disclosed in US-A-4,922,034.

The conversion conditions to be utilized in the peroxide decomposition zone may comprise, for example, a temperature of 100° to 200°C, a pressure of 550 to 3450 kPa (80 to 500 psia) and a space velocity of 0.5 to 20 volumes of feed per volume of catalyst per hour.

The effluent from the peroxide decomposition zone will typically comprise 95 to 99 wt.% of tertiary butyl alcohol and less than 0.1 wt.% of peroxide contaminants.

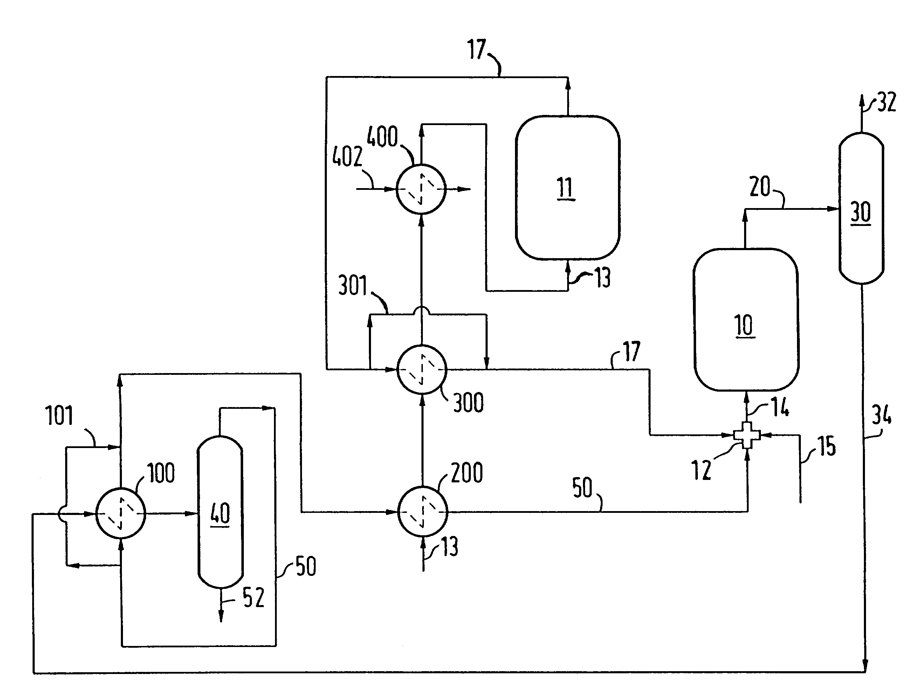

Turning now to the drawing, there is shown a schematic flow sheet illustrating a preferred method for the practice of the process of the present invention. In the drawing, conventional parts, such as valves, pumps, temperature control sensors, pressure sensors, heaters, coolers, flow control regulation apparatus, reflux condenses and reboilers have been omitted.

In accordance with the present invention, there is provided an etherification reaction zone 10 containing a bed of solid etherification catalyst. Any suitable catalyst may be used such as, for example, a solid resin etherification of the type described above, such as a strongly acidic ion exchange resin consisting essentially of sulfonated polystyrene crosslinked with divinyl benzene (e.g., Dowex®50, Nalcite®HCR, Amberlyst®15). As another example, the catalyst may be a fluorophosphoric acid-on-titania catalyst of the type disclosed in US-A-4,822,921 or a heteropoly acid such as 12-tungstophosphoric acid or 12-molybdophosphoric acid supported on an inert support such as titania.

When the tertiary butyl alcohol is prepared by the thermal or catalytic decomposition of tertiary butyl hydroperoxide, it will contain minor amounts of impurities such that, for example, the feedstock charged to the reaction zone 10 will typically contain the following components:

| ETHERIFICATION REACTION ZONE FEED MIXTURE | |
|---|---|
| Component | wt.% (approximate) |
| Methanol | 41.0 |
| TBA¹ | 47.0 |
| Acetone | 0.5 |
| 2-Propanol | 6.0 |
| MTBE² | 0.2 |
| DTBP³ | 0.1 |
| t-Butyl Formate | 0.1 |
| Water | 6.0 |

| | |
|---|---|
| ¹ Tertiary butyl alcohol | |
| ² Methyl tertiary butyl ether | |
| ³ Ditertiary butyl peroxide | |

The peroxides-contaminated tertiary butyl alcohol feedstock is initially charged by way of a tertiary butyl alcohol feed line 13 to a peroxides decomposition zone 11, such as, for example a zone 11 operated, (e.g.) at a temperature of 100° to 200°C., a pressure of 550 to 3450 kPa (80 to 500 psia) and a flow rate of 0.5 to 20, preferably 0.5 to 4, volumes of feedstock per reactor volume per hour to thereby provide a substantially peroxides-free tertiary butyl alcohol reaction product. The peroxide contaminants will be decomposed to form water and tertiary butyl alcohol, and trace amounts of other decomposition products such as acetone and methyl formate.

The peroxides-contaminated feedstock and the substantially peroxides-free reaction product discharged from the peroxide decomposition zone 11 will typically have compositions as follows:

| PEROXIDE DECOMPOSITION ZONE FEED AND PRODUCT | | |
|---|---|---|
| Component | Feed (wt.%) | Product (wt.%) |
| DTBP¹ | 0.87 | 0.02 |
| TBA² | 97.2 | 97.4 |
| Water | 0.1 | 0.02 |
| Other³ | 1.8 | 2.6 |

| | | |
|---|---|---|
| ¹ Ditertiary butyl peroxide | | |
| ² Tertiary butyl alcohol | | |
| ³ Includes acetone, tertiary butyl formate, isopropyl alcohol, etc. | | |

The substantially peroxides-free tertiary butyl alcohol reaction product is continuously discharged from the peroxides decomposition zone 11 by a discharge line 17 leading ultimately to a manifold 12. A methanol feed stream is continuously charged to the manifold 12 by a line 15, as is a recycle stream 50 containing recycle tertiary butyl alcohol and recycle methanol. The flow of methanol and tertiary butyl alcohol to the manifold 12 through the lines 15, 17 and 50 is regulated so that a molar excess of methanol is present in the line 14 leading to the etherification reaction zone 10, such as, for example, a molar ratio of 1.1 to 3 moles, preferably 2 moles, of methanol per mol of tertiary butyl alcohol.

Within the etherification reaction zone 10, the feed mixture is brought into contact with the bed of etherification catalyst, such as a sulfonic acid resin etherification catalyst under reaction conditions including a pressure of 205 to 3450 kPa (30 to 500 psia), and more preferably from 1380 to 2070 kPa (200 to 300 psia), a temperature of 30° to 200°C, and more preferably from 80° to 140°C, and still more preferably from 90° to 130°C. When the catalyst is a supported phosphorus acid-type catalyst, the reaction temperature may suitably be in the range of 150° to 190°C.

Contact time within the etherification reaction zone is suitably such that 0.5 to 20 volumes of feed mixture per volume of etherification catalyst per hour are fed to the etherification reaction zone 10 and, more preferably from 1 to 4 volumes of feed mixture per volume of etherification catalyst per hour.

Within the etherification reaction zone 10, methanol will exothermically react with the tertiary butyl alcohol to form methyl tertiary butyl ether which will be contained in a reaction product discharged from the etherification reaction zone 10 by way of a line 20 leading to a methyl tertiary butyl ether (MTBE) distillation zone 30.

As a specific example, when the solid etherification catalyst is a sulfonic acid resin such as Amberlyst^{®}15 and when the molar ratio of methanol to tertiary butyl alcohol in the feed mixture charged to the etherification reaction zone 10 by the line 14 is within the ratio of 2.0 moles of methanol per mole of tertiary butyl alcohol, and the reaction is conducted at a temperature of 100°C at a feed rate of 2.0 volumes of feed mixture per volume of catalyst per hour, the etherification reaction product typically may have the composition in part shown by the following table:

| ETHERIFICATION REACTION PRODUCT | |
|---|---|
| Component | % |
| Water | 14.0 |
| Methanol | 27.6 |
| Isobutylene | 3.0 |
| TBA¹ | 14.1 |
| MTBE² | 34.5 |
| Other³ | 6.8 |

| | |
|---|---|
| ¹ Tertiary butyl alcohol | |
| ² Methyl tertiary butyl ether | |
| ³ Includes the acetone, propanol, ditertiary butyl peroxide, tertiary butyl formate, etc. initially present in the tertiary butyl alcohol feedstock. | |

The etherification reaction product charged to the MTBE distillation zone 30 by way the charge line 20 is fractionated therein under distillation conditions including a liquid reflux temperature of 30° to 100°C, and more preferably 40° to 80°C, a reboiler temperature of 80° to 115°C, and more preferably from 95° to 105°C, and a pressure of 100 to 415 kPa (15 to 60 psia), the distillation condition being selected such that substantially all of the MTBE in the etherification reaction product 20 is taken overhead from the first distillation zone 30 by a line 32. As a consequence, the first distillation fraction 32 taken overhead from the distillation zone 30 will comprise substantially all of the isobutylene and substantially all of the methyl tertiary butyl ether and some of the methanol charged to the first distillation zone 30. The higher boiling distillation fraction 34 discharged from the MTBE distillation zone 30 will comprise methanol, tertiary butyl alcohol and water.

Typically, the first lower boiling distillation fraction 32 will comprise 6.5 wt.% isobutylene, 16.5 wt.% methanol and 75 wt.% MTBE and the higher boiling fraction 34 will comprise 37 wt.% methanol, 26.0 wt.% tertiary butyl alcohol, 26 wt.% water, 11 wt.% isopropanol and 0.5 wt.% of other components.

The higher boiling distillation fraction 34 discharged from the MTBE distillation zone 30 is charged to a recycle distillation zone 40 for the recovery of tertiary butyl alcohol and methanol and is fractionated under distillation conditions including a liquid reflux temperature of 35° to 170°C, and more preferably 140° to 150°C, and a reboiler temperature of 100° to 190°C, more preferably 170° to 180°C, and at a pressure of 100 to 1300 kPa (15 to 190 psia), and more preferably 760 to 1100 kPa (110 to 160 psia), into a lower boiling distillation fraction 50 discharged from the distillation zone 40 by a line 50 and a higher boiling water fraction 52 discharged from the distillation zone 40 by a line 52.

In accordance with the present invention, the higher boiling fraction 34 is heated to a desired distillation temperature of 115° to 135°C in a first by-pass indirect heat exchanger 100. This is accomplished by bringing the higher boiling fraction 34 into indirect heat exchange contact with a first portion of a lower boiling distillation fraction 50 in the first by-pass indirect heat exchanger 100 in order to raise the temperature of the higher boiling distillation fraction 34 to the desired level; the amount of lower boiling distillation fraction 50 charged to the first by-pass indirect heat exchanger 100 being sufficient to raise the temperature of the higher boiling distillation fraction 34 to the desired level and to simultaneously lower the temperature of the first portion of the lower boiling distillation fraction 50. The remaining portion of the lower boiling distillation fraction 50 flows around the first by-pass indirect heat exchanger 100 through by-pass line 101 and then rejoins the first portion to provide a reconstituted lower boiling fraction 50 having a desired lower intermediate temperature.

Thus, the higher boiling distillation fraction 34 may be charged to said first by-pass indirect contact heat exchanger 100 at a temperature of 100° to 120°C and heated in the by-pass indirect heat exchanger 100 to a temperature of 115° to 135°C and 40 to 60 vol.% of the lower boiling distillation recycle fraction 50 (the first portion thereof) may be charged to the first by-pass indirect contact heat exchanger 100 at a temperature of 140° to 160°C and cooled in the first by-pass indirect heat exchanger 100 by an amount sufficient to provide a reconstituted lower boiling distillation recycle fraction 50 having a predeterminable temperature within a predetermined temperature range of 125° to 145°C.

In the event that the temperature of the reconstituted lower boiling distillation recycle fraction 50 is to be changed, the percentage of the first portion of the lower boiling distillation recycle fraction 50 that is charged to the first by-pass indirect contact heat exchanger 100 will be changed to thereby regulate the temperature at which the higher boiling distillation fraction 34 exits the first by-pass indirect heat exchanger 100.

In accordance with the present invention, the reconstituted lower boiling distillation fraction 50 is next charged to an initial indirect contact heat exchanger 200 at a temperature of 125° to 145°C and cooled in said initial indirect heat exchanger 200 to a temperature of 90° to 110°C by indirect heat exchange contact with the peroxides-contaminated TBA feedstock 13; the peroxides contaminated TBA feedstock 13 being charged to the initial by-pass indirect contact heat exchanger 200 at a temperature of 40° to 60°C and heated in said initial indirect heat exchanger 200 to an initial temperature of 100° to 130°C.

Thereafter, the further cooled reconstituted lower boiling distillation fraction 50 is charged to the manifold 12 from which the charge line 14 leads to the etherification reaction zone 10.

The initially heated peroxides contaminated TBA feedstock 13 is next charged to a second indirect by-pass heat exchanger 300 where it is brought into indirect heat exchange contact with a first portion of the substantially peroxides-free tertiary butyl alcohol reaction product 17 discharged from the peroxides decomposition zone 11. The initially heated TBA feed stream 13 is heated in the second indirect by-pass heat exchanger 300 to an intermediate temperature of 150° to 170°C. The first portion of the substantially peroxides-free tertiary butyl alcohol reaction product 17 is charged to the second by-pass heat exchanger 300 at a temperature of 170° to 190°C and cooled in said second by-pass indirect heat exchanger 300 to a temperature of 125° to 145°C. The amount of the substantially peroxides-free tertiary butyl alcohol reaction product 17 charged to the second by-pass indirect heat exchanger 300 should be sufficient to raise the temperature of the initially heated peroxides contaminated TBA feedstock 13 to a desired level and to simultaneously lower the temperature of the first portion of the substantially peroxides-free tertiary butyl alcohol reaction product 17.

Thus, reconstituted substantially peroxides-free tertiary butyl alcohol reaction product 17 is charged to the second indirect by-pass heat exchanger 300 where it is brought into indirect heat exchange contact with 10 to 40 vol.% of the substantially peroxides-free tertiary butyl alcohol reaction product 17 (the first portion) discharged from the peroxides decomposition zone 11 and heated therein to the intermediate temperature of 150° to 170°C in the second by-pass indirect heat exchanger 300.

The remaining portion of the substantially peroxides-free tertiary butyl alcohol reaction product 17 flows around the second by-pass indirect heat exchanger 300 through by-pass line 301 and then rejoins the first portion to provide a reconstituted substantially peroxides-free tertiary butyl alcohol reaction product 17 having a desired lower intermediate temperature.

In the event that the temperature of the reconstituted substantially peroxides-free tertiary butyl alcohol reaction product 17 is to be changed, the percentage of the first portion of the substantially peroxides-free tertiary butyl alcohol reaction product 17 that is charged to the first by-pass indirect contact heat exchanger 100 will be changed to thereby regulate the temperature of the reconstituted substantially peroxides-free tertiary butyl alcohol reaction product 17.

The reconstituted substantially peroxides-free tertiary butyl alcohol reaction product 17 is then charged to the manifold 12 from which the charge line 14 leads to the etherification reaction zone 10.

The further heated peroxides contaminated TBA feedstock 13 is then charged to a final indirect contact heat exchanger 400 for indirect heat exchange contact with a heating medium (such as steam) charged by a line 402 to provide a finally heated peroxides-contaminated TBA feedstock having a temperature of 175° to 185°C. The finally heated peroxides-contaminated TBA feedstock is charged to peroxides decomposition reaction zone 11 under thermal peroxides decomposition reaction conditions including a temperature of 175° to 185°C, a pressure of 80 to 500 psia and a flow rate of 0.5 to 4 volumes of feedstock per reactor volume per hour to substantially completely decompose the peroxide contaminants to thereby form the second substantially peroxides-free TBA stream.

The substantially peroxides-free reaction product discharged from the peroxide decomposition zone 11 exits the peroxides decomposition reaction zone 11 by line 17.

The by-pass indirect heat exchangers 100 and 300 are useful in maintaining the temperature of the final charge stream at a temperature within a predetermined temperature range. An initial temperature for the final charge stream is established that is within the predetermined temperature range. If the temperature of the final charge stream changes during operations due to process upsets, distillation upsets, etc., the initial temperature or another temperature within the predetermined temperature range can be established by adjusting flow through the by-pass lines 101 and 301.

For example, in a representative operation, the temperature of the final charge stream can be reduced by 3.3°C (6°F) by reducing the by-pass flow through line 101 of indirect by-pass heat exchanger 100 from 50% to 0 and the temperature of the final charge stream can be reduced by 1.7°C (3°F) by reducing the by-pass flow through line 301 of indirect by-pass heat exchanger 300 from 30% to 0. Thus, in this situation, control of flow through the by-pass line 101 offers a wider range of temperature control than control of the flow through by-pass lined 301. However, the first by-pass indirect contact heat exchanger 100 is separated from the charge line 14 by initial indirect contact heat exchanger 200, so the impact on the temperature of the final charge stream resulting from a change in the flow through line 301 of the second by-pass indirect heat exchanger 300 will be experienced more rapidly than a change in the flow through the line 101.

Hence, the overall control strategy can rely on adjustment of the rate of flow through by-pass line 301 in order to more immediately compensate for a fluctuation in the temperature of the final charge stream and by then adjusting the rate of flow through the by-pass line 101 to more affirmatively reestablish the desired temperature of the final charge stream 14.

## Claims

1. A method for mixing three streams each of which has a predetermined flow rate and a temperature different from the temperature of the other streams in order to provide a final mixed stream having a predetermined flow rate and a desired predeterminable temperature within a predetermined temperature range which comprises:
a) flowing a first selected portion of a primary stream through a first by-pass indirect contact heat exchanger into indirect heat exchange contact with a flowing stream of a heat exchange liquid,
b) flowing a second selected portion of said primary stream through the by-pass of said first by-pass indirect contact heat exchanger,
c) mixing said first and second selected portions of said primary stream downstream of said first by-pass indirect contact heat exchanger to provide a reconstituted primary stream having a desired intermediate temperature,
d) flowing a first selected portion of a secondary stream through a second by-pass indirect contact heat exchanger into indirect heat exchange contact with a flowing stream of a heat exchange liquid,
e) flowing a second selected portion of said secondary stream through the by-pass of said second by-pass indirect contact heat exchanger,
f) mixing said first and second portions of said secondary stream downstream of said second by-pass indirect contact heat exchanger to provide a reconstituted secondary stream having a desired intermediate temperature,
g) mixing said reconstituted primary stream and said reconstituted secondary stream with a third stream to provide a final stream having a desired final predeterminable temperature within the predetermined temperature range, and
h) changing the portion of at least one of said primary stream or said secondary stream flowing through a by-pass of said first and second by-pass indirect contact heat exchangers in response to a change in the temperature of the final mixed stream by an amount sufficient to maintain said final mixed stream at a temperature within the predetermined temperature range.

2. A method as claimed in Claim 1 for preparing a charge stream for use in the preparation of methyl tertiary butyl ether (MTBE) from tertiary butyl alcohol (TBA) and methanol (MeOH), wherein a charge stream comprising MeOH and TBA is charged to a primary MTBE etherification reaction zone under etherification reaction conditions including a reaction temperature within a predetermined temperature range of 90° to 140°C, which comprises the steps of:
a) charging the etherification reaction product to a first MTBE distillation zone and separated therein into a first lighter distillation fraction comprising isobutylene, MeOH and MTBE and a second heavier distillation fraction comprising MeOH, TBA and water,
b) charging the second heavier distillation fraction to a recycle distillation column and distilling it therein to provide a lower boiling distillation recycle fraction having a temperature above the predetermined reaction temperature range,
c) charging a peroxides-contaminated TBA feedstock to a peroxides decomposition reaction zone to substantially completely decompose the peroxide contaminants to thereby form a high temperature substantially peroxides-free TBA feed stream having a temperature above said predetermined temperature range,
d) flowing said higher boiling distillation fraction from said MTBE distillation zone through a first by-pass indirect contact heat exchanger into indirect heat exchange contact with a flowing stream comprising a predetermined portion of said lower boiling distillation recycle fraction while flowing the remainder of said lower boiling distillation recycle fraction through the by-pass of said by-pass indirect contact heat exchanger, and recombining the said portions of said lower boiling distillation recycle fraction to form a reconstituted lower boiling distillation recycle fraction having a desired lower temperature,
e) flowing a selected portion of said high temperature substantially peroxides-free TBA feed stream through the by-pass of a second by-pass indirect contact heat exchanger and flowing the remainder of said high temperature substantially peroxides-free TBA feed stream through said second by-pass indirect contact heat exchanger in indirect heat exchange contact with said peroxides-contaminated TBA feedstock and recombining the said portions of said high temperature substantially peroxides-free TBA feed stream downstream of said second by-pass indirect contact heat exchanger to form a reconstituted second stream having a desired lower temperature,
f) mixing said reconstituted lower boiling distillation recycle fraction and said reconstituted second stream with a first MeOH feed stream to provide a final stream having a desired final predeterminable temperature within the predetermined temperature range, and
g) changing the portion of at least one of said lower boiling distillation recycle fraction or said high temperature substantially peroxides-free TBA feed stream flowing through a by-pass of said first and second by-pass indirect contact heat exchangers in response to a change in the temperature of said final stream by an amount sufficient to maintain said final stream at a temperature within the predetermined temperature range.
